# EUROPEAN PATENT SPECIFICATION

(11) **EP 1 244 447 B1**
(45) Date of publication and mention of the grant of the patent: **10.01.2007**
(21) Application number: 00992256.8
(22) Date of filing: 29.11.2000
(51) Int. Cl.: A61K 31/445, A61K 31/485

(54) **NOVEL METHODS AND COMPOSITIONS INVOLVING OPIOIDS AND ANTAGONISTS THEREOF**
NEUE VERFAHREN UND ZUBEREITUNGEN DIE OPIOIDEN UND DEREN ANTAGONISTEN ENTHALTEN
NOUVEAUX PROCEDES ET NOUVELLES COMPOSITIONS COMPRENANT DES OPIOIDES ET DES ANTAGONISTES D'OPIOIDES

(30) Priority: 29.11.1999 US 450806
(43) Date of publication of application: 02.10.2002
(73) Proprietor: Adolor Corporation, Exton, PA 19341-1127 (US)
(72) Inventor: FARRAR, John, J., Chester Springs, PA 19425 (US)
(74) Representative: W.P. Thompson & Co.
(86) International application number: PCT/US2000/042315
(87) International publication number: WO 2001/037785

(56) References cited:
- EP-A- 0 287 339
- US-A- 4 457 933
- US-A- 4 582 835
- US-A- 4 769 372
- US-A- 5 250 542
- WITTELS B ET AL: "OPIOID ANTAGONIST ADJUNCTS TO EPIDURAL MORPHINE FOR POSTCESAREAN ANALGESIA: MATERNAL OUTCOMES" ANESTHESIA AND ANALGESIA, WILLIAMS AND WILKINS, BALTIMORE, MD, US, vol. 77, no. 5, November 1993 (1993-11), pages 925-932, XP001095958 ISSN: 0003-2999
- GERAK L R ET AL: "CHANGES IN SENSITIVITY TO THE RAT-DECREASING EFFECTS OF OPIODIS IN PIGEONS TREATED ACUTELY OR CHRONICALLY WITH NALBUPHINE" BEHAVIOURAL PHARMACOLOGY, RAPID SCIENCE, PUBLISHERS, GB, vol. 5, no. 7, October 1996 (1996-10), pages 437-447, XP008005675 ISSN: 0955-8810
- GEAR R W ET AL: "ACTION OF NALOXONE ON GENDER-DEPENDENT ANALGESIC AND ANTIANALGESIC EFFECTS OF NALBUPHINE IN HUMANS" JOURNAL OF PAIN, SAUNDERS, PHILADELPHIA, PA, US, vol. 1, no. 2, 21 June 2000 (2000-06-21), pages 122-127, XP008005634 ISSN: 1526-5900
- VACCARINO A L ET AL: "Endogenous opiates: 1998." PEPTIDES. UNITED STATES DEC 1999, vol. 20, no. 12, December 1999 (1999-12), pages 1527-1574, XP002236928 ISSN: 0196-9781
- OSOL: 'Remington's pharmaceutical sciences', 1975 'Opiate analgesics', page 1098, XP001149582 15th Edition
- WINDHOLZ: 'The merck index, an encyclopedia of chemicals, drugs and biologicals', 21 July 1986 XP002942738 10th edition, no. 2423 * page 350 *
- FLESHMAN J.W. ET AL: 'Managing postoperative ileus: Evaluation of alvimopan in three phase II clinical studies', October 2002, 88TH ANNUAL CLINICAL CONGRESS
- 'Adolor targets pain/itch' WORLD PHARMACEUTICAL NEWS no. 2412, 12 February 1999,
- 'Adolor to Develop Copound to Treat Narcotic-Induced Constipation; In-licensed From Roberts Pharmaceuticals' PR NEWSWIRE 16 June 1998, page 0616NYTU019
- ZIMMERMANN ET AL DRUGS OF THE FUTURE vol. 19, no. 12, 1994, pages 1078 - 1083

## Description

### Field of the Invention

The present invention relates to novel compositions comprising opioids and opioid antagonists. More particularly, the present invention relates to novel compositions comprising opioids and peripheral mu opioid antagonist compounds.

### Background of the Invention

It is well known that opioid drugs target three types of endogenous opioid receptors (i.e., mu, delta and kappa receptors) in biological systems. Most opioids, such as morphine, are mu opioid agonists that are often used as analgesics for the treatment of severe pain due to their activation of mu opioid receptors in the brain and central nervous system (CNS). Opioid receptors are, however, not limited to the CNS, and may be found in other tissues throughout the body. A number of side effects of opioid drugs may be caused by activation of these peripheral receptors. Administration of mu opioid agonists often results in intestinal dysfunction due to the large number of receptors in the wall of the gut (Wittert, G., Hope, P. and Pyle, D., *Biochemical and Biophysical Research Communications* **1996**, 218, 877-881; Bagnol, D., Mansour, A., Akil, A. and Watson, S.J., *Neuroscience* **1997**, 81, 579-591). Specifically, opioids are generally known to cause nausea and vomiting as well as inhibition of normal propulsive gastrointestinal function in animals and man (Reisine, T., and Pasternak, G., *Goodman & Gilman 's The Pharmacological Basis of Therapeutics Ninth Edition* **1996**, 521-555) resulting in side effects such as, for example, constipation. It has been reported that acute nausea or vomiting may occur in up to about 33% of patients who receive oral narcotic analgesics and in up to about 80% of patients who receive injectable narcotics following surgery or trauma. This is due, at least in part, to direct effects of narcotics on the gastrointestinal (GI) tract.

Opioid-induced side effects, such as nausea, vomiting, and inhibited gastrointestinal propulsive activity remain serious problems for patients being administered opioid analgesics for both short term and long term pain management. Opioid antagonist compounds that do not readily cross the blood-brain barrier (peripherally acting drugs) have been tested for use in curbing opioid-induced side effects. For instance, the peripheral mu opioid antagonist compound methylnaltrexone and related compounds have been suggested for use in curbing opioid-induced side effects in patients. U.S. Patent Nos. 5,972,954, 5,102,887, 4,861,781, and 4,719,215 disclose the use of methylnaltrexone and related compounds in controlling opioid-induced pruritus, nausea, and/or vomiting. Additionally, methylnaltrexone has been shown to effectively reduce the incidence of opioid-induced nausea and pruritus as disclosed by Yuan, C.-S. et al. *Drug and Alcohol Dependence* **1998**, 52, 161. Similarly, U.S. Patent Nos. 5,250,542, 5,434,171, 5,159,081, and 5,270,328, disclose peripherally selective piperidine-N-alkylcarboxylate opioid antagonists as being useful for the treatment of the opioid side effects constipation, nausea or vomiting, as well as irritable bowel syndrome and idiopathic constipation.

It is frequently the case that drugs have undesirable side effects, and patients taking such drugs are often prescribed additional drugs for countering these side effects. Thus, patients may be required to take multiple doses of different drugs, causing inconvenience and possible administration of incorrect doses. It may therefore be desirable for multiple drugs to be combined as one dose in a fixed ratio for ease of administration. Given that nausea, vomiting, and inhibited gastrointestinal propulsive activity are common side effects of opioid analgesics that contribute to the discomfort of a patient receiving such therapy, a need for a specific and effective side effect-relieving remedy is present. As it is not readily evident to combine two or more drugs for simultaneous administration, due to the complex nature of drug interactions which are often undesirable and even fatal to the patient, it is desirable to identify drug formulations that contain compounds when taken simultaneously in pre-measured, fixed-dose forms, resulting in safe alternative means for administering multiple drugs. In the present invention, it has been found that opioid analgesics, with their common undesirable side effects, are optimal candidates for such formulations in combination with specific peripheral mu opioid antagonist compounds.

"Adolor targets pain/itch", in World Pharmaceutical News, published 12.02.99, issue no. 2912 discloses the use of ADL8-2698 (also known as alvimopan) For co-administration with opioid analgesics to prevent constipation without interfering with analgesic activity.

### Summary of the Invention

Accordingly, the present invention is directed to oral pharmaceutical compositions comprising 15 to 200 mg of an opioid, 0.1 to 4 mg as a peripheral mu opioid antagonist, and a pharmaceutically acceptable carrier, wherein said opioid antagonist compound is a compound of formula (II), as herein defined.

These and other aspects of the invention will become more apparent from the following detailed description.

### Brief Description of the Drawing

Figure 1 is a graphical representation of studies on the inhibition of the slowing of gut motility employing compositions according to an embodiment of the present invention.
Figures 2A and 2B are graphical representations of studies on the inhibition of nausea and vomiting employing compositions according to an embodiment of the present invention.

### Detailed Description of the Invention

"Side effect" refers to a consequence other than the one(s) for which an agent or measure is used, as the adverse effects produced by a drug, especially on a tissue or organ system other then the one sought to be benefitted by its administration. In the case, for example, of opioids, the term "side effect" may preferably refer to such conditions as, for example, constipation, nausea and/or vomiting.

"Effective amount" refers to an amount of a compound as described herein that may be therapeutically effective to inhibit, prevent or treat the symptoms of particular disease, disorder or side effect. Such diseases, disorders and side effects include, but are not limited to, those pathological conditions associated with the administration of opioids (for example, in connection with the treatment and/or prevention of pain), wherein the treatment or prevention comprises, for example, inhibiting the activity thereof by contacting cells, tissues or receptors with compounds of the present invention. Thus, for example, the term "effective amount", when used in connection with opioids, for example, for the treatment of pain, refers to the treatment and/or prevention of the painful condition. The term "effective amount", when used in connection with peripheral mu opioid antagonist compounds, refers to the treatment and/or prevention of side effects typically associated with opioids including, for example, such side effects as constipation, nausea and/or vomiting.

"In combination with", "combination therapy" and "combination products" refer, in certain embodiments, to the concurrent administration to a patient of opioids and peripheral mu opioid antagonists, including, for example, the compounds of formula (I). When administered in combination, each component may be administered at the same time or sequentially in any order at different points in time. Thus, each component may be administered separately but sufficiently closely in time so as to provide the desired therapeutic effect.

"Dosage unit" refers to physically discrete units suited as unitary dosages for the particular individual to be treated. Each unit may contain a predetermined quantity of active compound(s) calculated to produce the desired therapeutic effect(s) in association with the required pharmaceutical carrier. The specification for the dosage unit forms of the invention may be dictated by (a) the unique characteristics of the active compound(s) and the particular therapeutic effect(s) to be achieved, and (b) the limitations inherent in the art of compounding such active compound(s).

"Pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

"Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

Certain acidic or basic compounds of the present invention may exist as zwitterions. All forms of the compounds, including free acid, free base and zwitterions, are contemplated to be within the scope of the present invention.

"Patient" refers to animals, including mammals, preferably humans.

The present invention is directed to pharmaceutical compositions involving opioid compounds. As discussed above, such opioid compounds may be useful, for example, in the treatment and/or prevention of pain. However, as also discussed above, undesirable side effects including, for example, constipation, nausea and vomiting, as well as other side effects, may frequently occur in patients receiving opioid compounds. By virtue of the compositions of the present invention, effective and desirable inhibition of undesirable side effects that may be associated with opioid compounds may be advantageously achieved. Accordingly, combination compositions, where opioids are combined or co-administered with suitable peripheral mu opioid antagonist compounds, may afford an efficacy advantage over the compounds and agents alone.

In this connection, as discussed above, patients are often administered opioids for the treatment, for example, of painful conditions. However, as noted above, undesirable side effects such as, for example, constipation, nausea and/or vomiting, may result from opioid administration. These undesirable side effects may act as a limiting factor in connection with the amount of opioid that may be administered to the patient. That is, the amount of opioid capable of being administered to the patient may be limited due to the undesired occurrence of the aforementioned side effects. The limited amounts of opioid that may be administered to a patient may, in turn, result in a disadvantageously diminished degree of pain alleviation. The present combination compositions may be used to advantageously increase the amount of opioid administered to a patient, thereby obtaining enhanced pain alleviation, while reducing, minimizing and/or avoiding undesirable side effects that may be associated with the opioid. The peripheral mu opioid antagonist employed in the compositions of the present invention has substantially no central nervous system activity and, accordingly, does not affect the pain killing efficacy of the opioid.

While not intending to be bound by any theory or theories of operation, it is contemplated that opioid side effects, such as constipation, vomiting and nausea, may result from undesirable interaction of the opioid with peripheral mu receptors. Administration of a mu opioid antagonist according to the present invention may block interaction of the opioid compounds with the mu receptors, thereby preventing and/or inhibiting the side effects.

A wide variety of opioids are available which may be suitable for use in the present compositions of the invention. Generally speaking, it is only necessary that the opioid provide the desired effect (for example, pain alleviation), and be capable of being incorporated into the present combination products (discussed in detail below). In preferred embodiments, the present compositions may involve an opioid which is selected from alfentanil, buprenorphine, butorphanol, codeine, dezocine, dihydrocodeine, fentanyl, hydrocodone, hydromorphone, levorphanol, meperidine (pethidine), methadone, morphine, nalbuphine, oxycodone, oxymorphone, pentazocine, propiram, propoxyphene, sufentanil and/or tramadol. More preferably, the opioid is selected from morphine, codeine, oxycodone, hydrocodone, dihydrocodeine, propoxyphene, fentanyl and/or tramadol.

The opioid component of the present compositions may further include one or more other active ingredients that may be conventionally employed in analgesic and/or cough-cold-antitussive combination products. Such conventional ingredients include, for example, aspirin, acetaminophen, phenylpropanolamine, phenylephrine, chlorpheniramine, caffeine, and/or guaifenesin. Typical or conventional ingredients that may be included in the opioid component are described, for example, in the *Physicians' Desk Reference,* 1999.

In addition, the opioid component may further include one or more compounds that may be designed to enhance the analgesic potency of the opioid and/or to reduce analgesic tolerance development. Such compounds include, for example, dextromethorphan or other NMDA antagonists (Mao, M. J. et al., *Pain* **1996**, 67,361), L-364,718 and other CCK antagonists (Dourish, C.T. et al., *Eur J Pharmacol* **1988***, 147,* 469), NOS inhibitors (Bhargava, H.N. et al., *Neuropeptides* **1996**, *30,* 219), PKC inhibitors (Bilsky, E.J. et al., *J Pharmacol Exp Ther* **1996**, 277,484), and dynorphin antagonists or antisera (Nichols, M.L. et al., *Pain* **1997**, *69,* 317).

Other opioids, optional conventional opioid components, and optional compounds for enhancing the analgesic potency of the opioid and/or for reducing analgesic tolerance development, that may be employed in the compositions of the present invention, in addition to those exemplified above, would be readily apparent to one of ordinary skill in the art, once armed with the teachings of the present disclosure.

Compounds of formula (II) for use in the present invention hone the following formula

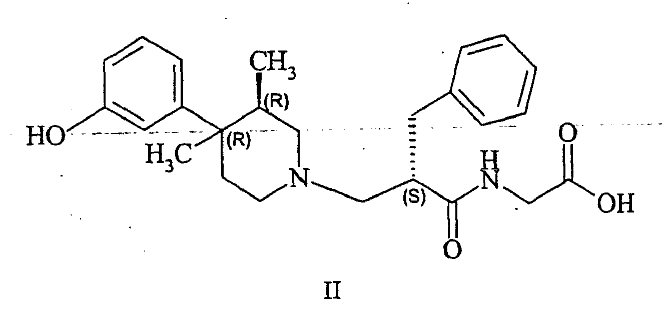

The compound of formula (II) has low solubility in water except at low or high pH conditions. Zwitterionic character may be inherent to the compound, and may impart desirable properties such as poor systemic absorption and sustained local affect on the gut following oral administration.

The compounds employed in the present invention may be prepared in a number of ways well known to those skilled in the art. The compounds can be synthesized, for example, by the methods described below, or variations thereon as appreciated by the skilled artisan. All processes disclosed in association with the present invention are contemplated to be practiced on any scale, including milligram, gram, multigram, kilogram, multikilogram or commercial industrial scale.

As will be readily understood, functional groups present may contain protecting groups during the course of synthesis. Protecting groups are known *per se* as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Preferred protecting groups include the benzyloxycarbonyl group and the tert-butyloxycarbonyl group. Other preferred protecting groups that may be employed in accordance with the present invention may be described in Greene, T.W. and Wuts, P.G.M., *Protective Groups in Organic Synthesis* 2d. Ed., Wiley & Sons, 1991.

Piperidine-N-alkylcarboxylate compounds according to the present invention may be synthesized employing methods taught, for example, in U.S. Patent Nos. 5,250,542, 5,434,171, 5,159,081, and 5,270,328.

For example, the 3-substituted-4-methyl-4-(3-hydroxy- or alkanoyloxyphenyl)piperidine derivatives employed as starting materials in the synthesis of the present compounds may be prepared by the general procedure taught in U.S. Patent No. 4,115,400 and U.S. Patent No. 4,891,379.

The starting material for the synthesis of compounds described herein, (3R,4R)-4-(3-hydroxypheny)-3,4-dimethylpiperidine, may be prepared by the procedures described in U.S. Patent No. 4,581,456. but adjusted as described such that the β-stereochemistry is preferred.

The first step of the process may involves the formation of the 3-alkoxyphenyllithium reagent by reacting 3-alkoxybromobenzene with an alkyllithium reagent. This reaction may be performed under inert conditions and in the presence of a suitable non-reactive solvent such as dry diethyl ether or preferably dry tetrahydrofuran. Preferred alkyllithium reagents used in this process are n-butyllithium, and especially sec-butyllithium. Generally, approximately an equimolar to slight excess of alkyllithium reagent may be added to the reaction mixture. The reaction may be conducted at a temperature of from about -20°C and about -100°C, more preferably from about -50°C to about -55°C.

Once the 3-alkoxyphenyllithium reagent has formed, approximately an equimolar quantity of a 1-alkyl-4-piperidone may be added to the mixture while maintaining the temperature between -20°C and -100°C. The reaction is typically complete after about 1 to 24 hours. At this point, the reaction mixture may be allowed to gradually warm to room temperature. The product may be isolated by the addition to the reaction mixture of a saturated sodium chloride solution to quench any residual lithium reagent. The organic layer may be separated and further purified if desired to provide the appropriate 1-alkyl-4-(3-alkoxyphenyl)piperidinol derivative.

The dehydration of the 4-phenylpiperidinol prepared above may be accomplished with a strong acid according to well known procedures. While dehydration occurs in various amounts with any one of several strong acids such as hydrochloric acid, hydrobromic acid, and the like, dehydration is preferably conducted with phosphoric acid, or especially p-toluenesulfonic acid in toluene or benzene. This reaction may be typically conducted under reflux conditions, more generally from about 50°C and 150°C. The product thus formed may be isolated by basifying an acidic aqueous solution of the salt form of the product and extracting the aqueous solution with a suitable water immiscible solvent. The resulting residue following evaporation can then be further purified if desired.

The 1-alkyl-4-methyl-4-(3-alkoxyphenyl)tetrahydropyridine derivatives may be prepared by a metalloenamine alkylation. This reaction is preferably conducted with n-butyllithium in tetrahydrofuran (THF) under an inert atmosphere, such as nitrogen or argon. Generally, a slight excess of n-butyllithium may be added to a stirring solution of the 1-alkyl-4-(3-alkoxyphenyl)-tetrahydropyridine in THF cooled to a temperature in the range of from about -50°C to about 0°C, more preferably from about -20°C to -10°C. This mixture may be stirred for approximately 10 to 30 minutes followed by the addition of approximately from 1.0 to 1.5 equivalents of methyl halide to the solution while maintaining the temperature of the reaction mixture below 0°C. After about 5 to 60 minutes, water may be added to the reaction mixture and the organic phase may be collected. The product can be purified according to standard procedures, but the crude product is preferably purified by either distilling it under vacuum or slurrying it in a mixture of hexane:ethyl acetate (65:35, v:v) and silica gel for about two hours. According to the latter procedure, the product may be then isolated by filtration followed by evaporating the filtrate under reduced pressure.

The next step in the process may involve the application of the Mannich reaction of aminomethylation to non-conjugated, endocyclic enamines. This reaction is preferably carried out by combining from about 1.2 to 2.0 equivalents of aqueous formaldehyde and about 1.3 to 2.0 equivalents of a suitable secondary amine in a suitable solvent. While water may be the preferred solvent, other non-nucleophilic solvents, such as acetone and acetonitrile can also be employed in this reaction. The pH of this solution may be adjusted to approximately 3.0 to 4.0 with an acid that provides a non-nucleophilic anion. Examples of such acids include sulfuric acid, the sulfonic acids such as methanesulfonic acid and p-toluenesulfonic acid, phosphoric acid, and tetrafluoroboric acid, with sulfuric acid being preferred. To this solution may be added one equivalent of a 1-alkyl-4-methyl-4-(3-alkoxyphenyl)tetrahydropyridine, typically dissolved in aqueous sulfuric acid, and the pH of the solution may be readjusted with the non-nucleophilic acid or a suitable secondary amine. The pH is preferably maintained in the range of from about 1.0 to 5.0, with a pH of about 3.0 to 3.5 being more preferred during the reaction. The reaction is substantially complete after about 1 to 4 hours, more typically about 2 hours, when conducted at a temperature in the range of from about 50°C to about 80°C, more preferably about 70°C. The reaction may then be cooled to approximately 30°C, and added to a sodium hydroxide solution. This solution may then be extracted with a water immiscible organic solvent, such as hexane or ethyl acetate, and the organic phase, following thorough washing with water to remove any residual formaldehyde, may be evaporated to dryness under reduced pressure.

The next step of the process may involve the catalytic hydrogenation of the prepared 1-alkyl-4-methyl-4-(3-alkoxyphenyl)-3-tetrahydropyridinemethanamine to the corresponding trans-1-alkyl-3,4-dimethyl-4-(3-alkoxyphenyl)piperidine. This reaction actually occurs in two steps. The first step is the hydrogenolysis reaction wherein the exo C-N bond is reductively cleaved to generate the 3-methyltetrahydropyridine. In the second step, the 2,3-double bond in the tetrahydropyridine ring is reduced to afford the desired piperidine ring.

Reduction of the enamine double bond introduced the crucial relative stereochemistry at the 3 and 4 carbon atoms of the piperidine ring. The reduction generally does not occur with complete stereoselectivity. The catalysts employed in the process may be chosen from among the various palladium and preferably platinum catalysts.

The catalytic hydrogenation step of the process is preferably conducted in an acidic reaction medium. Suitable solvents for use in the process include the alcohols, such as methanol or ethanol, as well as ethyl acetate, tetrahydrofuran, toluene, hexane, and the like.

Proper stereochemical outcome may be dependent on the quantity of catalyst employed. The quantity of catalyst required to produce the desired stereochemical result may be dependent upon the purity of the starting materials in regard to the presence or absence of various catalyst poisons.

The hydrogen pressure in the reaction vessel may not be critical but can be in the range of from about 5 to 200 psi. Concentration of the starting material by volume is preferably around 20 mL of liquid per gram of starting material, although an increased or decreased concentration of the starting material can also be employed. Under the - conditions specified herein, the length of time for the catalytic hydrogenation may not be critical because of the inability for over-reduction of the molecule. While the reaction can continue for up to 24 hours or longer, it may not be necessary to continue the reduction conditions after the uptake of the theoretical two moles of hydrogen. The product may then be isolated by filtering the reaction mixture for example through infusorial earth, and evaporating the filtrate to dryness under reduced pressure. Further purification of the product thus isolated may not be necessary and preferably the diastereomeric mixture may be carried directly on to the following reaction.

The alkyl substituent may be removed from the 1-position of the piperidine ring by standard dealkylation procedures. Preferably, a chloroformate derivative, especially the vinyl or phenyl derivatives, may be employed and removed with acid. Next, the prepared alkoxy compound may be dealkylated to the corresponding phenol. This reaction may be generally carried out by reacting the compound in a 48% aqueous hydrobromic acid solution. This reaction may be substantially complete after about 30 minutes to 24 hours when conducted at a temperature of from about 50°C to about 150°C, more preferably at the reflux temperature of the reaction mixture. The mixture may then be worked up by cooling the solution, followed by neutralization with base to an approximate pH of 8. This aqueous solution may be extracted with a water immiscible organic solvent. The residue following evaporation of the organic phase may then be used directly in the following step.

The compounds employed as starting materials to the compounds of the invention can also be prepared by brominating the 1-alkyl-4-methyl-4-(3-alkoxyphenyl)-3-tetrahydropyridinemethanamine at the 3-position, lithiating the bromo compound thus prepared, and reacting the lithiated intermediate with a methylhalide, such as methyl bromide to provide the corresponding 1-alkyl-3,4-dimethyl-4-(3-alkoxyphenyl)tetrahydropyridinemethanamine. This compound may then be reduced and converted to the starting material as indicated above.

Intermediates can be prepared by reacting a 3,4-alkyl-substituted-4-(3-hydroxyphenyl)piperidine with a compound of the formula LCH₂(CH₂)ₙ₋₁CHR³C(O)E where L is a leaving group such as chlorine, bromine or iodine, E is a carboxylic acid, ester or amide, and R³ and n are as defined hereinabove. Preferably L may be chlorine and the reaction is carried out in the presence of a base to alkylate the piperidine nitrogen. For example 4-chloro-2-cyclohexylbutanoic acid, ethyl ester can be contacted with (3R,4R)-4-(3-hydroxyphenyl)-3,4-dimethylpiperidine to provide 4-[(3R,4R)-4-(3-hydroxyphenyl)-3,4-dimethyl-1-piperidine]butanoic acid, ethyl ester. Although the ester of the carboxylic acid may be preferred, the free acid itself or an amide of the carboxylic acid may be used.

In alternative synthesis, the substituted piperidine can be contacted with a methylene alkyl ester to alkylate the piperidine nitrogen. For example, 2-methylene-3-phenylproponic acid, ethyl ester can be contacted with a desired piperidine to provide 2-benzyl-3-piperidinepropanoic acid ethyl ester.

Another synthetic route can involve the reaction of a substituted piperidine with a haloalkylnitrile. The nitrile group of the resulting piperidine alkylnitrile can be hydrolyzed to the corresponding carboxylic acid.

With each of the synthetic routes, the resulting ester or carboxylic acid can be reacted with an amine or alcohol to provide modified chemical structures. In the preparation of amides, the piperidine-carboxylic acid or -carboxylic acid ester may be reacted with an amine in the presence of a coupling agent such as dicyclohexylcarbodiimide, boric acid, borane-trimethylamine, and the like. Esters can be prepared by contacting the piperidine-carboxylic acid with the appropriate alcohol in the presence of a coupling agent such as p-toluenesulfonic acid, boron trifluoride etherate or N,N'-carbonyldiimidazole. Alternatively, the piperidine-carboxylic acid chloride can be prepared using a reagent such as thionyl chloride, phosphorus trichloride, phosphorus pentachloride and the like. This acyl chloride can be reacted with the appropriate amine or alcohol to provide the corresponding amide or ester.

The compounds employed in the present invention including, for example, opioid and peripheral mu opioid antagonist compounds, may be administered orally. The compounds may be administered by any conventional means available for use in conjunction with pharmaceuticals, either as individual therapeutic agents or in a combination of therapeutic agents. For example, they may be administered as the sole active agents in a pharmaceutical composition, or they can be used in combination with other therapeutically active ingredients.

The compounds are combined with a pharmaceutical carrier selected on the basis of the chosen route of administration and standard pharmaceutical practice as described, for example, in *Remington's Pharmaceutical Sciences* (Mack Pub. Co., Easton, PA, 1980).

The active compounds may be orally administered, with an inert diluent or with an assimilable edible carrier, or may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like.

The tablets, troches, pills, capsules and the like may also contain one or more of the following: a binder, such as gum tragacanth, acacia, corn starch or gelatin; an excipient, such as dicalcium phosphate; a disintegrating agent, such as com starch, potato starch, alginic acid and the like; a lubricant, such as magnesium stearate; a sweetening agent such as sucrose, lactose or saccharin; or a flavoring agent, such as peppermint, oil of wintergreen or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring, such as cherry or orange flavor. Of course, any material used in preparing any dosage unit form is preferably pharmaceutically pure and substantially non-toxic in the amounts employed. In addition, the active compound may be incorporated into sustained-release preparations and formulations.

The combination products of this invention, such as pharmaceutical compositions comprising opioids in combination with a peripheral mu opioid antagonist compound formula (II), may be in any oral dosage form, such as those described herein.

The combination products of the invention are formulated together, in a single dosage form (that is, combined together in one capsule, tablet, powder, or liquid, etc.). With regard to a typical dosage form of this type of combination product, such as a tablet, the opioid compounds (*e.g.*, morphine) generally is present in an amount of 15 to 200 milligrams, and the peripheral mu opioid antagonists in an amount of 0.1 to 4 milligrams.

When provided as a single dosage form, the potential exists for a chemical interaction between the combined active ingredients (for example, an opioid and a peripheral mu opioid antagonist compound). For this reason, the preferred dosage forms of the combination products of this invention are formulated such that although the active ingredients are combined in a single dosage form, the physical contact between the active ingredients is minimized (that is, reduced).

In order to minimize contact, one embodiment of this invention where the product is orally administered provides for a combination product wherein one active ingredient is enteric coated. By enteric coating one or more of the active ingredients, it is possible not only to minimize the contact between the combined active ingredients, but also, it is possible to control the release of one of these components in the gastrointestinal tract such that one of these components is not released in the stomach but rather is released in the intestines. Another embodiment of this invention where oral administration is desired provides for a combination product wherein one of the active ingredients is coated with a sustained-release material which effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low-viscosity grade of hydroxypropyl methylcellulose (HPMC) or other appropriate materials as known in the art, in order to further separate the active components. The polymer coating serves to form an additional barrier to interaction with the other component.

Dosage forms of the combination products of the present invention wherein one active ingredient is enteric coated can be in the form of tablets such that the enteric coated component and the other active ingredient are blended together and then compressed into a tablet or such that the enteric coated component is compressed into one tablet layer and the other active ingredient is compressed into an additional layer. Optionally, in order to further separate the two layers, one or more placebo layers may be present such that the placebo layer is between the layers of active ingredients. In addition, dosage forms of the present invention can be in the form of capsules wherein one active ingredient is compressed into a tablet or in the form of a plurality of microtablets, particles, granules or non-perils, which are then enteric coated. These enteric coated microtablets, particles, granules or non-perils are then placed into a capsule or compressed into a capsule along with a granulation of the other active ingredient.

These as well as other ways of minimizing contact between the components of combination products of the present invention, whether administered in a single dosage form or administered in separate forms but at the same time by the same manner, will be readily apparent to those skilled in the art, once armed with the present disclosure.

### Background EXAMPLES

The invention is further demonstrated in the following background examples. All of the background examples are actual examples. The examples are for purposes of illustration and are not intended to limit the scope of the present invention.

### Background Example 1

This example is directed to *in vivo* experiments in mice which demonstrate the effectiveness of the combination products of the present invention.

In a mouse model of opioid-induced constipation (measured by the charcoal meal transit time), the compound of formula (II), orally administered, prevented acute morphine-induced constipation. A 3 mg/kg oral dose had a duration of action between 8 and 24 hours. Additional studies showed that the compound of formula (II) was even more potent in reversing morphine-induced constipation in chronic morphine treated mice. This establishes that the compound of formula (II) is a gut-selective and peripherally-selective mu antagonist compound. In addition, it is orally effective in preventing or reversing morphine-induced constipation in mice.

The following examples are directed to *in vivo* experiments in humans which demonstrates the effectiveness of the combination methods and products of the present invention.

### Background Example 2

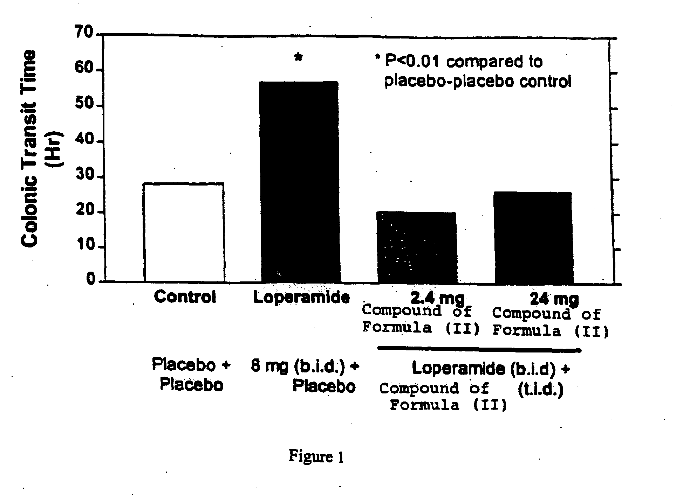

A clinical study in man was an 8 subject multiple crossover study of the effects of oral pre-treatment with placebo, 2.4 mg or 24 mg t.i.d. of the compound of formula (II) on slowing of gut motility induced with 8 mg of b.i.d. of oral loperamide (a peripheral mu opioid agonist). Both doses of the compound of formula (II) prevented loperamide-induced slowing of gut motility as shown in the graph illustrated in Figure 1. The graph presents the effects of 2.4 or 24 mg of the compound of formula (II) on colonic transit time (in hours) following administration of loperamide. The loperamide dose was constant in the three treatment groups. Since both doses of the compound of formula (II) completely prevented loperamide-induced increased colonic transit time, the effective dose range of the compound of formula (II) may be well below the lowest dose (2.4 mg t.i.d.) evaluated in the study.

### Background Example 3

A Phase I study in 20 healthy volunteers demonstrated that a 4 mg oral dose of the compound of formula (II) blocked the effect of intravenous morphine sulfate on upper gastrointestinal motility (P<0.01). The compound of formula (II) also showed a trend toward antagonizing morphine-induced nausea (P=0.07) indicating that the compound of formula (II) may provide additional benefits to patients experiencing common adverse side effects from morphine or other opioids.

### Background Example 4

A Phase I study in 11 volunteers demonstrated that a 3 mg oral dose of the compound of formula (II) administered three times daily for 4 days blocked the inhibition of gastrointestinal transit produced by oral sustained-release morphin (MS Contin®, 30 mg twice daily) without antagonizing MS Contin® effects on pupil size. Pupil size was used as a surrogate measure of the morphine's analgesic activity.

### Background Example 5

A double-blind Phase II clinical study in 24 young healthy patients undergoing third molar extraction dental surgery showed that the compound of formula (II) (4 mg total oral dose) did not antagonize analgesia or pupil constriction produced by intravenous morphine sulfate. No patients were withdrawn for adverse effects.

### Background Example 6

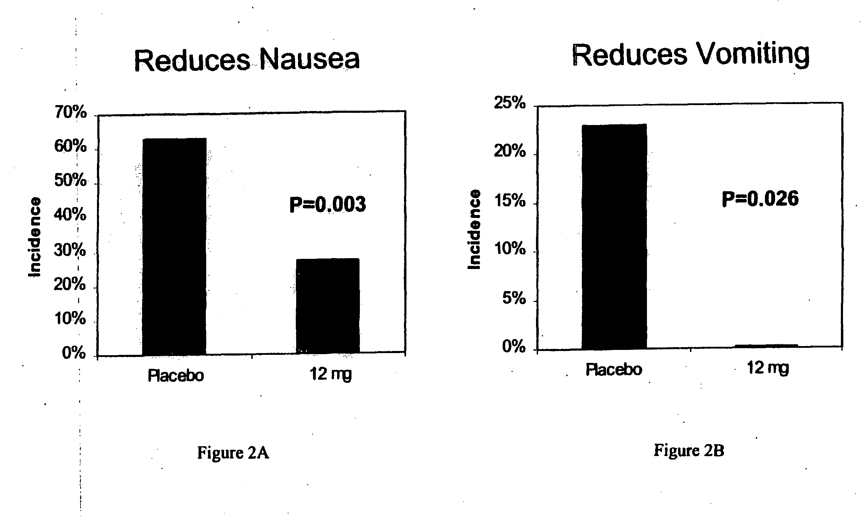

A 78 patient Phase II clinical study was conducted which compared two doses (2 mg and 12 mg) of the compound of formula (II) versus placebo in patients undergoing partial colectomy or simple or radical hysterectomy surgical procedures. All patients in this clinical study received morphine or meperidine infusions to treat postoperative pain. Oral doses of compound (II) or placebo were administered to block postsurgical opioid effects, including postoperative nausea and vomiting. Results of this study comparing patients receiving 12 mg of compound (II) and placebo are depicted graphically in Figures 2A and 2B.

The intensity of nausea was evaluated by patients on a 100-point visual analog scale (VAS) with VAS=0 being no nausea and VAS=100 being the worst nausea that a patient could imagine. The highest VAS nausea score (worst nausea) recorded for each patient was computed and the distributions of these maximum values were compared among the treatment groups. Nearly 40% of the patients receiving 12 mg per day of the compound of formula (II) exhibited no nausea (highest VAS score = 0), compared to approximately 25% of the 2 mg per day group and just over 10% of the placebo group. The overall treatment differences in the distributions were significant when compared using a Kruskal-Wallis test (P=0.0184). The improved outcomes observed in the 12 mg per day dose group are evident in the pairwise comparisons based on the Wilcoxon rank sum tests. The 12 mg per day dose group had results that were statistically significantly improved compared to the placebo dose (P=0.0072). These results are further supported by noting that only 27% of the 12 mg per day dose group reported VAS scores over 20, compared to 63% of the placebo group and 67% of the 2 mg dose group (P=0.003 using the Mantel-Haenszel test for linear trend). No patients experienced serious adverse side ) effects in this trial that were judged by the clinical investigator to be related to the activity of the compound of formula (II). None of the patients receiving the compound of formula (II) experienced a reduction in postoperative pain control, indicating the selectivity of the compound of formula (II) for blocking opioid nausea and vomiting without blocking analgesia.

These results demonstrate that the compound of formula (II) blocked the adverse gastrointestinal effects of morphine or other narcotic analgesics that were used for post-surgical pain relief.

## Claims

1. An oral pharmaceutical composition comprising 15 to 200mg of an opioid, 0.1 to 4mg of a peripheral mu opioid antagonist compound; and a pharmaceutically acceptable carrier, wherein said opioid antagonist compound is a compound of formula (II):

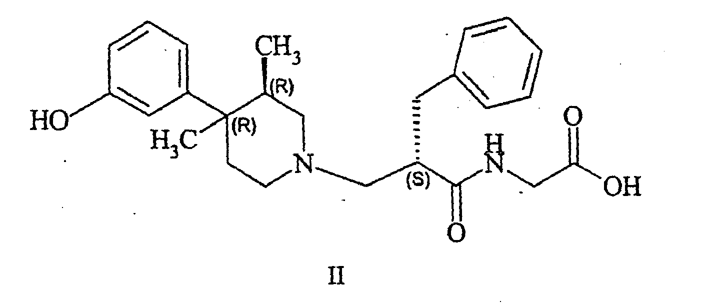

2. A composition according to claim 1, wherein said opioid is selected from alfentanil, buprenorphine, butorphanol, codeine, dezocine, dihydrocodeine, fentanyl, hydrocodone, hydromorphone, levorphanol, meperidine (pethidine), methadone, morphine, nalbuphine, oxycodone, oxymorphone, pentazocine, propiram, propoxyphene, sufentanil or tramadol.

3. A composition according to claim 2, wherein said opioid is selected from morphine or meperidine.

4. A composition according to any preceding claim which is in a single dosage unit form.

5. A composition according to claim 1 which is a tablet.

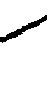

## Patentansprüche

1. Eine orale pharmazeutische Rezeptur, bestehend aus 15 bis 200mg eines Opioids, 0,1 bis 4mg einer peripheren µ-Opioid-Antagonisten-Verbindung und einem pharmazeutisch akzeptierbaren Carrier, worin diese Opioid-Antagonisten-Verbindung eine Verbindung von Formel (II) ist.

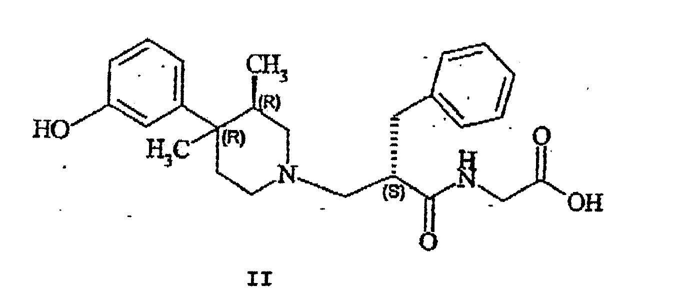

2. Eine Rezeptur gemäß Anspruch 1, worin dieses Opioid ausgewählt ist aus Alfentanil, Buprenorphin, Butorphanol, Codein, Dezocin, Dihydrocodein, Fentanyl, Hydrocodon, Hydromorphon, Levorphanol, Meperidin (Pethidin), Methadon, Morphin, Nalbuphin, Oxycodon, Oxymorphon, Pentazocin, Propiram, Propoxyphen, Sufentanil und/oder Tramadol.

3. Eine Rezeptur gemäß Anspruch 2, worin dieses Opioid ausgewählt ist aus Morphin und Meperidin.

4. Eine Rezeptur gemäß irgendeinem der vorstehenden Ansprüche, welche in einer Einzeldosierungs-Einheitsform ist.

5. Eine Rezeptur gemäß Anspruch 1, welche eine Tablette ist.

## Revendications

1. Composition pharmaceutique comprenant de 15 à 200 mg d'un opioïde, 0,1 à 4 mg d'un composé d'antagoniste d'opioïde périphérique mu, et un vecteur pharmaceutiquement acceptable, dans laquelle ledit composé d'antagoniste d'opioïde est un composé de formule (II) :

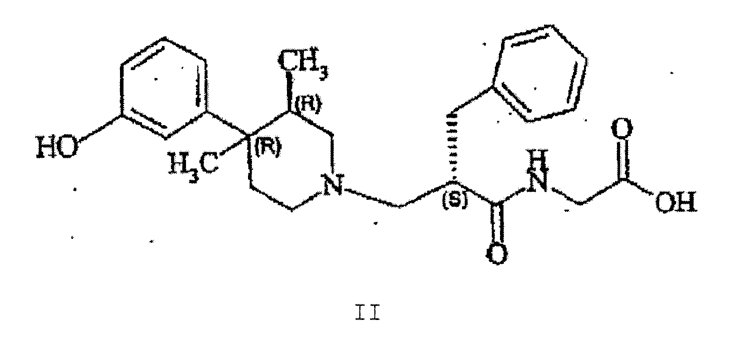

2. Composition selon la revendication 1, dans laquelle ledit opioïde est sélectionné parmi l'alfentanil, le buprenorphine, le butorphanol, la codéine, la dézocine, la dihydrocodéine, le fentanyl, l'hydrocodone, l'hydromorphone, le levorphanol, la mépéridine (péthidine), la méthadone, la morphine, la nalbuphine, l'oxycodone, l'oxymorphone, la pentazocine, le propiram, le propoxyphène, le sufentanil ou le tramadol.

3. Composition selon la revendication 2, dans laquelle ledit opioïde est sélectionné parmi la morphine ou la mépéridine.

4. Composition selon l'une quelconque des revendications précédentes qui se présente sous la forme d'une dose unique.

5. Composition selon la revendication 1 qui est un comprimé.
